# EUROPEAN PATENT SPECIFICATION

(11) **EP 0 390 252 B1**
(45) Date of publication and mention of the grant of the patent: **31.07.1996**
(21) Application number: 90200624.6
(22) Date of filing: 15.03.1990
(51) Int. Cl.: C12P 21/02, A61K 38/18, A61K 38/19

(54) **Purification of recombinant human interleukin-3**
Reinigung von rekombinantem, menschlichem Interleukin-3
Purification de l'interleukine-3 recombinante humaine

(30) Priority: 15.03.1989 EP 89200660; 25.07.1989 EP 89201967
(43) Date of publication of application: 03.10.1990
(73) Proprietor: GIST-BROCADES N.V., NL-2600 MA Delft (NL)
(72) Inventor: Persoon, Nicolaas Ludovicus Maria, lNL-2331 NA Leiden (NL); Van Leen, Robert Willem, NL-2914 TN Nieuwerkerk a/d IJsel (NL)
(74) Representative: Matulewicz, Emil Rudolf Antonius, Dr.

(56) References cited:
- WO-A-88/00598
- WO-A-88/04691
- WO-A-88/05469
- US-A- 4 419 446
- THE JOURNAL OF IMMUNOLOGY, vol. 129, no. 6, December 1982, American Association of Immunologists, US; J.N. IHLE et al., pp. 2431-2436/
- Biotechnology vol. 9, 1991, pp 47-52/

## Description

### Technical Field

The present invention relates to the purification of human interleukin-3 (hIL-3) and muteins thereof. The purification method, an inventive combination of chromatographic steps, yields a substantially pure product.

### Background Art

The clinical utility of human IL-3 (hIL-3) is not only dependent on its inherent characteristics but also on its availability and the lack of contaminants. The literature pertaining to the purification of murine, gibbon and human IL-3 is briefly reviewed here.

The mature murine T-cell enzyme marker 20α-hydroxy-steroid dehydrogenase (20αSDH) was found to be inducible in vitro. The factor responsible for this was partially purified from splenic lymphocytes by Ihle et al., J. Immunol. (1981), 129, 2184-2189. It was distinct from other known lymphokines in both its biochemical and functional properties. Ihle et al. proposed the term interleukin-3 ("IL-3") for this factor. The purification by Sephadex G-100 and DEAE cellulose chromatography resulted in a 9000 fold purification, yet the final preparation still contained multiple proteins.

An improved purification procedure was presented by Ihle et al., J. Immunol. (1982), 129, 2431-2436, WEHI-3 cells which constitutively produced murine IL-3 (mIL-3), were used. Here, through the extension of the earlier procedure with hydroxylappatite and reverse-phase high performance liquid chromatography (HPLC), the final product could be obtained 1,800,000 fold purified (their Table I). This product was claimed to be homogeneous.

Miyajima et al., Gene (1987), 58, 273-281, used the silkworm Bombyx mori, and an insect baculovirus vector for high-level expression and secretion of murine IL-3. Purification of mIL-3 from tissue culture medium was carried out by sequential passage through DEAE-Sephadex, ACA 54 and C8 reverse-phase column chromatography. To obtain separation of three species of mIL-3 (18, 20 and 22 kDaA) a second C8 reverse-phase column was necessary. The different species are due to differential glycosylation, since N-glycanase treatment yielded one final band of 15 kDa.

Ziltener et al., J. Biol. Chem. (1988), 263, 14511-14517 described the isolation of multiple glycosylated forms of IL-3 by affinity purification. The observed micro-heterogeneity was dependent on the source (activated T-cells, WEHI-3B cells or COS 7 cells).

All of the above procedures described the purification of murine IL-3. In spite of the observed similarity of murine and human IL-3 with respect to their proliferative action on haematopoietic progenitor cells, the structural homology between both proteins is rather low (28% at the amino acid level). The structural difference leads to a difference in specificity as illustrated by the total absence of reactivity of the human protein on murine cells (and vice versa). Based on their specific amino acid composition, both proteins require different methods of purification.

The purification of gibbon and human IL-3, which show a structural homology of 93% (at the amino acid level), is disclosed in several patent publications. WO 88/00598 describes the isolation of a partially synthetic hIL-3 from the inclusion bodies of E. coli cells. The cells are first disrupted by two passages through a French press, and the inclusion bodies are isolated by centrifugation in a sucrose step gradient. This reference also describes three procedures for purifying a human or gibbon IL-3-like polypeptide from COS cell conditioned medium. In all cases a one-column process is used: either ion-exchange or a lentil lectin column or reversed-phase HPLC. The maximum purity obtained for gibbon IL3 as determined with automated Edman degradation was 98%. However, this is by no means a technique by which the purity of proteins can be accurately determined.

WO 88/05469 describes the purification of human IL-3 from yeast strains by single or sequential reversed-phase HPLC steps. The number of HPLC runs used is not specified. It is indicated that if required additional steps can be used. No tests determining the final purity of the hIL-3 were described. This clearly suggests that no homogeneous product was obtained.

It can be concluded that no specific methods have been disclosed so far for the purification of hIL-3 and compounds with a similar activity. Therefore there is still a need for substantially pure hIL-3 which can be used therapeutically and for a method of preparing such a substantially pure product in a high yield. Preferably this method should be easy to scale up.

The cloning and expression of human IL-3 has been described in the following references.

A DNA sequence encoding hIL-3 has been identified by Yang et al., Cell (1986), 47, 3-10, who isolated the hIL-3 gene using a cDNA encoding gibbon IL-3 as a probe and disclosed the sequence of the exons of the human gene.

WO 88/00598 describes that this human gene is transcribed in eukaryotic cells, wherein the RNA is spliced. After isolating the RNA, cDNA is made and the hIL-3 cDNA is identified by hybridization with gibbon IL-3 cDNA.

Dorssers et al., Gene (1987), 55, 115-124, isolated a cDNA encoding hIL-3 by hybridizing with a mouse IL-3 cDNA. WO 88/04691 points out that this hybridization was possible due to the unexpectedly high degree of homology between the 3′ noncoding regions of the human and the mouse IL-3 gene. After cloning the cDNA, the hIL-3 was subsequently expressed in a variety of host cells including E. coli, Bacilli, yeasts and tissue cultures. Generally expression products were biologically active. This patent application is the first to describe the presence of Pro at position 8 of the mature protein.

In WO 88/05469 the isolation of a cDNA encoding hIL-3 is described using a synthetic DNA as a probe which is derived from the genomic sequence described by Yang et al. (opt. cit.). The obtained cDNA sequence, however, lacks the information for two amino acids (nos. 44 and 45, or 45 and 46, which is arbitrary since the amino acid bordering either deletion is a GAC encoding Asp). Nonetheless, the culture supernatant of a yeast transformant carrying said cDNA sequence in an expression cassette, encoding mature hIL-3 fused to an N-terminal "flag" of 8 amino acids, is said to show IL-3 activity in a human bone marrow proliferation assay.

In WO 88/06161 the isolation of a hIL-3 cDNA sequence is disclosed using as a probe a synthetic DNA derived from the genomic sequence described by Yang et al. (opt. cit.). Also described is the construction of a completely synthetic hIL-3 coding sequence and the construction of two muteins Ile² and Leu¹³¹. There is no mention of biological activity. Furthermore, it was apparently presumed that hIL-3 contains 132 amino acids, starting at the N-terminus with Pro¹-Met²-, whereas it is generally accepted that hIL-3 is 133 amino acids long and has as the N-terminus Ala¹-Pro²-Met³-.

It is noteworthy that Yang et al. disclose a Ser residue at position 8 of the mature hIL-3, whereas all other references indicate the presence of a Pro at this position.

In EP-A-285429 the isolation of a human cell line producing a factor with mast cell growth factor activity is described, which is incorrectly named IL-3. It is well accepted that hIL-3 has no proliferating effect on mouse marrow cells, whereas the biochemically uncharacterized factor described in this reference is able to stimulate such cells to form mast cells. It can therefore not be identical to hIL-3.

To get a better understanding of one of the expression vector systems used to produce hIL-3, the following background information concerning the bovine papillomavirus is essential.

BPV-1 is one of at least six bovine papillomaviruses and is associated with cutaneous fibropapillomas in cattle. These viruses can readily transform a variety of rodent cells in culture. The molecularly cloned bovine papillomavirus DNA as well as a cloned 69% subgenomic fragment are very efficient in inducing transformed foci. Transformed cells contain multiple copies (10 to 120 per cell) of the viral DNA, as unintegrated molecules (Law et al., Proc. Natl. Acad. Sci. USA (1981), 78, 2727-2731). The genetics of bovine papillomavirus type I have extensively been studied (reviewed by Lambert et al., Annu. Rev. Genet. (1988), 22, 235-258). The BPV-1 genome is a circular, 7946 base-pair, double-stranded DNA molecule. The transcription is complicated because of the presence of multiple promoters, splice sites and differential production of RNA species. The activities of some of the promoters are under tight control of transcriptional enhancers.

BPV-1 or the 69% subgenomic fragment (BamHI-HindIII) has been used extensively for the expression cloning of a variety of genes in different cloning systems. EP-A-198386 describes the expression of gamma-interferon in C127 mouse cells. In EP-A-105141 the use of the BPV vector is described for the expression of hepatitis B surface antigen (HBsAg) in vertebrate cell lines e.g. NIH 3T3, LTK- mouse fibroblasts and African green monkey kidney cells. The use of BPV-1 as a part of cloning vehicles is disclosed in U.S. Patent No. 4,419,446.

### Summary of the Invention

According to one aspect of the present invention a protein having primate IL-3 activity is provided which is purified to homogeneity.

The invention provides a method for purifying a protein having IL-3 activity to homogeneity, by an initial stage of hydrophobic interaction, followed by ion-exchange chromatography and gel filtration. This method is particularly useful for hIL-3 obtained by recombinant prokaryotic and eukaryotic expression systems. These and other aspects will be further outlined in the detailed description hereinafter.

### Brief Description of the Drawings

Fig. 1 shows the amino acid sequence and the nucleotide sequence of the expression cassette of hIL-3 that is used in B. licheniformis. The arrow indicates the starting point of the mature protein.

Fig. 2 shows the elution profile of the hydrophobic interaction chromatography of hIL-3 from Bacillus licheniformis T9 on Fraktogel® TSK-butyl 650C (dxh = 25x8cm). The hIL-3 containing fractions are indicated with a horizontal bar.

Fig. 3 shows the elution profile of the anion-exchange chromatography (first time) of hIL-3 from Bacillus licheniformis T9 on Q-Sepharose® Fast Flow (dxh = 10x11cm).

Fig. 4 shows the elution profile of the anion-exchange chromatography (second time) of hIL-3 from Bacillus licheniformis T9 on Q-Sepharose® Fast Flow (dxh = 5x90cm).

Fig. 5 shows the elution profile of the gel filtration chromatography of hIL-3 from Bacillus licheniformis T9 on Sephacryl® S100 HR (dxh = 5x90cm).

Fig. 6 shows the number of leucocytes and thrombocytes in chimpanzee blood after subcutaneous injection of 30 µg/kg IL-3, during day 0 to 6.

### Detailed Description

The term "human interleukin-3" and its equivalents "humanIL-3" or "hIL-3" in this specification are used to indicate both human interleukin-3 and any protein with human IL-3-like activity. In a broad sence "IL-3" is used as an equivalent for primate IL-3. Although the present description specifically aims at human IL-3, it will be obvious to those skilled in the art that primate IL-3, having a high degree of homology with human IL-3, can be purified using the same method. Preferably the homology is at least 70% at the amino acid level, more preferably it is at least 90%. For example, the mature rhesus monkey (Macaca mulatta) IL-3 has 80.5% identity at the amino acid level with the human IL-3 sequence.

Other naturally occurring variants of IL-3 are also candidates for the purification procedure disclosed in this invention. Non-naturally occurring mutants can also be purified as long as the identity is not to low (e.g. 70%). Non-natural mutants are mutated proteins having retained IL-3 activity. Mutations include additions, deletions and substitutions. Proteins modified in such more or less obvious ways are also included.

"Recombinant" should be interpreted as produced by microbial organisms or by cell cultures that normally do not produce this protein or, at least, do not produce this protein in an amount which allows industrial application. Microbial organisms include bacteria, yeasts and fungi. Furthermore, tissue culture cells can also be used these are especially important when appropriate glycosylation is required.

The invention provides an efficient method for purifying recombinant human IL-3 to homogeneity. This method comprises a series of steps including hydrophobic interaction, ion-exchange chromatography and gel filtration. A specific combination of the said methods surprisingly proved to be extremely powerful for obtaining substantially pure hIL-3. Moreover, this purification method results in a pyrogen-free product. The method is very suitable for upscaling. Which is a big advantage since large volumes of the fermentation broth, containing hIL-3 after secretion from the cells, have to be purified from contaminating components of the growth medium. The method is particularly useful for the purification of hIL-3 produced by transformed hosts, both prokaryotic and eukaryotic.

In principle the above purification methods can be used in an arbitrary order. Every mentioned method as such would already result in a considerable purification. The inventors, however, are the first to show the specific combination described hereinafter.

Preferably the initial stage of the purification is hydrophobic interaction. This step, is unusual and therefore it is surprising that it turns out to be very effective for the purpose of this invention. Presumably, it causes a selective separation on the basis of the structure of the protein itself. In this step contaminants like other proteins, medium components and color are substantially removed.

Preferably ion-exchange chromatography is used as a second step. hIL-3 is found in the run-through fractions separated from most of the contaminating proteins. This step also results in the removal of residual color. Furthermore the amount of DNA and endotoxin is considerably reduced when anion-exchange chromatography is used.

When hIL-3 degradation products are present in the starting material to be purified, for example from Bacillus licheniformis, these proteins can be advantageously separated from native hIL-3 by means of a second anion-exchange chromatography step. Preferably Q Sepharose® Fast-Flow is used to obtain the desired result. Chromatography on hydroxylapatite and chromatofocussing on pBE94 are examples of other efficient techniques for the separation of hIL-3 degradation products.

As a final purification step gel filtration is advantageously used. In this procedure low molecular weight proteins or aggregation products are separated from the mature IL-3.

The hydrophobic interaction is advantageously performed using a TSK-butyl or octyl Sepharose® column, from which hIL-3 can be eluted for example with gradients of either (NH₄)₂SO₄ in Tris-HCl or ethylene glycol.

The removal of contaminating proteins (and other substances) by ion-exchange chromatography may require the use of columns such as, for example, TSK-DEAE, Q Sepharose® Fast-Flow or TSK-CM.

Finally, gel filtration using, preferably, Biogel® A or Sephacryl® S100 HR is an excellent final step in the purification of unglycosylated hIL-3.

The starting point for the purification can be either intracellular hIL-3 or hIL-3 contained in the fermentation broth.

The purification of intracellular hIL-3 (as illustrated herein after expression of hIL-3 in E. coli) is made much easier when the protein is contained in so-called inclusion bodies. The first step would then be the isolation of these inclusion bodies which contain the product in a relatively pure form and at a high concentration. After solubilization hIL-3 can be further purified by chromatography.

The purification of hIL-3 contained within the fermentation broth (as illustrated herein by the use of B. licheniformis, yeast and eukaryotic cell cultures, respectively) starts with obtaining cell-free medium.

To illustrate the general applicability of the present method to the purification of recombinantly obtained hIL-3, several expression vectors have been made. These vectors are examples of a wide range of vectors that can be used to obtain expression of hIL-3 in bacteria, fungi, yeasts and other eukaryotic cells. The construction of these expression vectors is described in general terms hereinafter.

In WO 88/04691 several ways for the production of hIL-3 are disclosed. The production by heterologous gene expression according to the present invention is on the one hand based on the combination of the perfect fusion of host signal sequences to mature hIL-3 coding sequences, in conjunction with strong promoters in Bacillus, and on the other hand on the use of strong promoter/enhancer combinations and mRNA stabilizing sequences on BPV-1 derived vectors in either C127, FR3T3 and CHO cells.

All elements used for expression of hIL-3 in Bacillus species have been described in WO 88/04691. However, optimal combinations of promoters, signal sequences and mature hIL-3 coding sequences were found by rearrangement of the different genetic elements. For proper secretion of hIL-3 by Bacillus species a perfect junction between α-amylase signal sequence and hIL-3 coding sequence was found to be crucial. Already using the σ43 promoter (as described in WO 88/04691) hIL-3 could be found in the culture medium, but only after inclusion of the strong α-amylase or HpaII promoter in the expression plasmid high level expression was obtained.

WO 88/04691 also describes expression of active hIL-3 by C127 cells. However, expression levels are relatively low when using the pLB4/BPV vector. The use of the combination of the Moloney Murine Sarcoma Virus (MSV) enhancer and mouse metallothionein I promoter, or the Human Cytomegalovirus enhancer/promoter, instead of the SV40 enhancer/promoter as present in pLB4/BPV, results in high expression levels of hIL-3 by mammalian cells.

Replacement of the hIL-3 cDNA sequence by the genomic hIL-3 sequence has a beneficial effect on the steady state level of hIL-3 mRNA and on the production of hIL-3 in mammalian cells.

A further increase in mRNA level can be achieved using BPV containing vectors. As mentioned in the description, the BPV-1 genome is a circular, 7946 base-pair, double-stranded DNA molecule. The transcription is complicated because of the presence of multiple promoters, splice sites and differential production of RNA species. The activities of some of the promoters are under tight control of transcriptional enhancers. The so-called E2 (= early) ORF is very important in this respect. The full-length E2 ORF encodes a transactivating protein (E2-ta) which can stimulate transcription of the early genes.

This protein consists of two conserved domains, the amino-terminal domain (which has transactivating activity) and the carboxy-terminal domain (which has both DNA-binding and dimer formation activities). The E2 ORF encodes a second regulatory protein, the E2 transcriptional repressor (E2-tr), which is an amino-terminally truncated form of the E2-ta protein. E2-tr is encoded by another mRNA, whereby the translational initiation codon is an E2 ORF internal ATG-codon. By mutating said start codon the production of E2-tr (the repressor protein) is clearly abolished. Without this repressor activity the transcripton of the early genes is considerably increased leading to an increase in plasmid copy number. This results in an increase of the transcription of the cloned gene (the amount of mRNA encoding the cloned gene increases) and thus in a higher protein production.

Two new cell lines are provided, derived from FR3T3 and CHO which may be used in the present invention. FR3T3 derived cell lines which were established by using a new expression vector with MSV enhancer/MT promoter, already showed an increase in hIL-3 production with respect to the previously disclosed C127-pLB4/BPV stable cell lines (WO 88/04691). It has been found that when this new expression vector is used to establish stable cell lines from C127 cells, a more than 20-fold increase can be obtained. These latter cell lines allow efficient production of hIL-3 with mammalian (complex) type glycosylation.

Furthermore, stable CHO cell lines that may be used in the present invention are provided. The use of CHO cells in combination with BPV vectors, for the production of pharmaceutical proteins, has not been described before. Production of hIL-3 with these cell lines equalled the best known production system. A further advantage of the use of CHO cells may be the ease with which these cells can be adapted to growth in suspension and then mass-cultured in large reactors. Furthermore, the disclosed broad host-range of BPV-vectors facilitates the choice of a cell line depending on the desired glycosylation.

Interestingly, hIL-3 from Kluyveromyces lactis obtained after purification on Fractogel® TSK-CM 650M showed a low but distinct binding affinity for heparin-Sepharose® as was detected by SDS-PAGE and immunoblotting. The affinity of hIL-3 for heparin may indicate a possible binding in vivo to similar matrices (the glycosaminoglycans, for instance) that are present within the human bone marrow stroma. Binding of hIL-3 to such a matrix may be part of its physiological function.

The presence of heparin-like and/or glycosaminoglycan-like bindingsite(s) on growth factors or other proteins could be an important mechanism for the in vivo targeting of such proteins to specific matrices within the bone marrow stroma or elsewhere in the body where their action is required. The development of second generation proteins with new or better binding sites for the above mentioned matrices could become important in formulating and targeting of pharmaceutical proteins such as hIL-3.

Human IL-3 has been defined extensively with respect to its biological properties in WO 88/04691 (opt. cit.). This reference also shows that some modifications of the IL-3 protein such as N-terminal extension, deletion of the Ala¹ or Ala¹-Pro² do not abolish biological activity of the protein. However, although it was argued that parts of the hIL-3 protein can be deleted or altered without a deleterious effect on its function, it can also be envisioned that hIL-3 like proteins with altered amino acid sequence have altered effects on target cells. Such molecules may either function as agonist or antagonist. For both classes of proteins clinical applications can be envisioned. Several of these hIL-3 like proteins retained their biological function, whereas other molecules have lost this function. The former proteins may still serve as agonist and might show properties beneficial in clinical use such as enhanced stability, better binding to the IL-3 receptor etcetera, whereas the latter proteins may serve as antagonist e.g. when binding of such a protein to the IL-3 receptor does not result in signal transduction.

The following examples are offered by way of illustration and not by way of limitation.

### Example 1

### Expression of Human IL-3 Bacillus sp.

In order to obtain an improved Bacillus expression vector for human IL-3 a derivative of plasmid pGB/IL-317 (WO 88/04691) was made. The latter plasmid contains downstream of the strong α-amylase promoter, in a 5' to 3' direction, coding information for the Bacillus α-amylase signal sequence - extra amino acids - the IL-3 signal sequence - mature IL-3 and the 3' end of the amylase gene.

First a perfect junction between the DNA sequence encoding the amylase signal sequence and the DNA sequence encoding mature human IL-3 was made in the E. coli-Bacillus shuttle-plasmid pGB/IL-311 (WO 88/04691). This plasmid was cleaved with restriction enzymes SalI and ClaI.

The small fragment containing the information for the human IL-3 signal sequence was exchanged with a synthetic SalI-ClaI fragment containing the coding information for the α-amylase signal sequence fused to the IL-3 coding sequence. The resulting plasmid pGB/IL-321 was transformed to B. licheniformis T9. Transformants synthesized only small amounts of IL-3, but the protein was secreted into the medium.

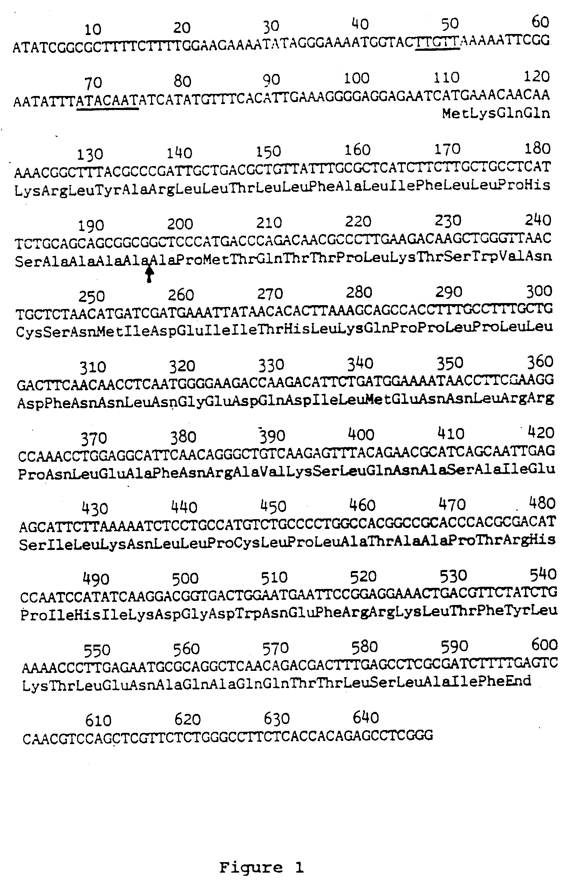

The small PstI-ClaI fragment from pGB/IL-321 was isolated and inserted into the PstI-ClaI cleaved plasmid pGB/IL-317, thereby exchanging the DNA sequence encoding the extra amino acids and the IL-3 signal sequence with the perfect junction of α-amylase signal sequence and mature IL-3 coding sequence. The resulting plasmid, which was designated pGB/IL-322, gives rise to high production of IL-3 by B. licheniformis T9 transformants. More than 95% of the protein is secreted into the culture medium. N-terminal sequencing of the purified protein showed that the IL-3 synthesized by these transformants has the correct amino-terminus (Ala¹-Pro²-Met³-). Figure 1 shows the complete amino acid sequence and the nucleotide sequence of the hIL-3 expression cassette as present in pGB/IL-322.

Another new expression vector pGB/IL-326 was constructed as follows: the HindIII-SalI fragment (HindIII end filled-in using Klenow polymerase) from pGB/IL-311 was cloned into pTZ18R (Pharmacia) cleaved with SalI and SmaI, resulting in vector pGB/IL-323. The DNA encoding the IL-3 signal sequence was exchanged with a synthetic DNA fragment encoding the Bacillus α-amylase signal sequence. In this synthetic piece of DNA the ATG start codon is preceded by the sequence CAT resulting in a cleavage site for the restriction enzyme NdeI. The SphI- KnpI fragment from this plasmid pGB/IL-324, containing α-amylase signal sequence - mature IL-3 - amylase terminator, was then cloned into pBAH3 cleaved with SphI and KpnI (pBAH3 is a derivative of pBHA1 (WO 88/04691) lacking the PstI site in the Ap^{R}-gene). The resulting plasmid pGB/IL-325 was cleaved with NdeI and religated, thereby fusing the so-called HpaII promoter with the α-amylase signal sequence - mature IL-3 encoding sequence. The resulting plasmid pGB/IL-326 was transformed to B. licheniformis T9. Transformants produced high amounts of IL-3 of which more than 95% was secreted into the culture medium.

The following steps were taken to obtain pGB/IL-341. The insert of pGB/IL-341 encodes a C-terminally truncated IL-3 (consisting of 129 amino acids). The 3' noncoding IL-3 cDNA and the α-amylase structural sequence between the IL-3 stopcodon in the cDNA and the α-amylase terminator were deleted from pGB/IL-324 by looping-out, using the following oligonucleotide:

5' TGA GCC TGC CGA TCT TTT GAC TGC AGT
AGA AGA GCA GAG AGG ACG G 3'

This oligonucleotide also introduced a XhoI site immediately downstream of the IL-3 stopcodon. The resulting plasmid was designated pGB/IL-337. The NdeI-KpnI fragment from pGB/IL-337 was cloned into the NdeI-KpnI cleaved E.coli vector pTZ18RN. pTZ18RN is pTZ18R modified immediately upstream of the lacZ ATG startcodon to introduce an NdeI site, using an oligonucleotide with the following sequence:

5' CAG GAA ACA CAT ATG ACC ATG ATT 3'

The resulting plasmid, in which the lacZ promoter is followed immediately by the sequence encoding the Bacillus α-amylase signal peptide precisely fused to mature IL-3, is designated pGB/IL-340. In this vector the IL-3 coding sequence was trimmed by looping out using an oligonucleotide with the following sequence:

5' CTC AAC AGA CGA CTT TGA GCT GAC TCG AGT AGA AGA
GCA GAG 3'

The resulting plasmid was called pGB/IL-340/1455. This E.coli plasmid thus encodes only the amino acids 1-129 of hIL-3.

A Bacillus expression vector for this mutein was constructed by replacing the PstI-HindIII fragment in pGB/IL-322 by the PstI-HindIII fragment of pGB/IL-340/1455. The resulting plasmid pGB/IL-341 gives rise to high production of the 129 amino acid IL-3 mutein by B.licheniformis T9 transformants.

IL-3 produced by B. licheniformis T9 transformants obtained with the expression vectors pGB/IL-322, pGB/IL-326 and pGB/IL-341 has the correct amino terminal sequence, was unglycosylated and showed high biological activity both in AML and human bone marrow assays.

### Example 2

### Expression of Human IL-3 by Mammalian Cells

An expression vector pGB/IL-328 with elements similar to those in p8-4 of Sarver et al. ("Papilloma viruses: Molecular and Clinical Aspects", 515-527 (1985), Alan R. Liss Inc.) was constructed. This vector contains a pML2 sequence (BamHI- ClaI, 2623 bp), the MSV enhancer fused to the mouse MT promoter (this fusion is described by Sarver et al., opt. cit.), IL-3 cDNA, the SV40 polyadenylation signal (as in Sarver et al., opt. cit.) and the complete BPV-1 genome.

To obtain the desired hIL-3 expression vector as a first step the hIL-3 encoding AVaII-AvaI fragment from pLB4 (with AvaI site filled in by Klenow polymerase) was cloned into EcoRI-SmaI cleaved pTZ18R (Pharmacia), together with a synthetic DNA fragment composed of the following two oligonucleotides:

                    10          20          30
        5'-AATTCAGATC   TAAAAATGAG   CCGCCTGCCC
GTCCTGCTCC  TGCTCCAACT  CCTG-3'
        40          50

                    10          20          30
5'-GACCAGGAGT  TGGAGCAGGA  GCAGGACGGG
CAGGCGGCTC  ATTTTTAGAT  CTG-3'
        40          50

The resulting plasmid, pGB/IL-327, was cleaved with BglII and BamHI, the hIL-3 encoding DNA fragment was isolated and cloned into the unique BglII site downstream of the MSV/mMT, enhancer/promoter described above. In the final hIL-3 expression vector pGB/IL-328 the BglII recognition sequence is followed by 4 A-residues after which the ATG initiation codon is placed. By using the AvaI site in the 3' non-coding region of the cDNA sequence for construction of the new expression vector the ATTTA repeats which have been implicated in mRNA instability (Shaw and Kamen, Cell (1986), 46, 659-667) are not included in pGB/IL-328. After introduction of pGB/IL-328 into C127 cells by conventional and previously published methods (e.g. WO 88/04691) stable cell lines were established which produce high levels of hIL-3. These cell lines have a more than 20-fold higher production level of hIL-3 than previously described stable cell lines derived from C127 cells carrying pLB4/BPV (WO 88/04691).

pGB/IL-328 was also introduced into FR3T3 cells (Seif and Cuzin, Virology (1977), 52, 721-728) using the method described above. Stable cell lines could also be established although with lower frequency. The obtained cell lines showed lower hIL-3 productivity than the C127 cell lines transformed with pGB/IL-328.

pGB/IL-328 was also introduced into CHO cells (Wood, R.D. and Burki, J.H., Mutat. Res. (1984), 95, 505) using the method described earlier. Since CHO cells do not form foci upon transfection of a vector carrying the BPV-1 sequences a co-transfection with pSV2neo (Southern, P.J. and Berg, P., J. Mol. Appl. Genet. (1982), 1, 327-341) was performed in a ratio of 10:1, (pSV2neo being the minor component), enabling selection for G418 resistance. G418-resistant colonies were isolated and tested for IL-3 production. A significant amount of these clones produced hIL-3. Subsequently, a single cell cloning procedure was carried out in order to establish stable cell lines. Several cell lines were established with production levels equivalent and better than the level of the best producing C127-pGB/IL-328 lines. This unexpected result has no precedent in the prior art.

The cytomegalovirus IE enhancer/promoter combination was used for expression of hI-3. The 815 bp Bal-SphI fragment from plasmid pES (Boom, Z. et al, J. Virol. (1986), 58, 851-859) was cloned into SmaI-SphI cleaved pTZ18R (Pharmacia). Subsequently a BglII site was introduced just downstream of the transcription initiation site using site-directed mutagenesis with the following oligonucleotide:

5' GAG CTC GTT TAG TGA ACC GTC AGA TCT CCT GGA
GAC GCC ATC CAC GCT GTT T 3'

An expression vector, pGB/IL-329, was constructed containing the pML2 sequence from pGB/IL-328 (BamHI-EcoRI), the CMV enhancer/promoter (EcoRI-BglII) and the SV40 polyadenylation signal (BglII-BamHI) as in Sarver et al., opt. cit. The final IL-3 expression construct pGB/IL-330 was made by introduction of the IL-3 encoding BamHI-BglII fragment from pGB/IL-327 into the unique BglII site of pGB/IL-329, followed by introduction of the BamHI cleaved BPV-1 genome into the unique BamHI site of this expression vector.

After introduction of pGB/IL-330 into C127 cells stable cell lines were established which produced high levels of IL-3. It is true that the expression is 2-4 times lower than with the pGB/IL-327 construct, but pGB/IL-330 gives rise to mRNA with an extremely short 5' untranslated region of 12 nucleotides. The introduction of a larger 5' untranslated region may facilitate translation, thereby enhancing the production level.

pGB/IL-330 was also introduced into CHO cells (Wood, R.D. and Burki, J.H., Mutat. Res. (1984), 95, 505) using the co-transfection method described above. G418-resistant colonies were picked and tested for hIL-3 production. Stable cell lines were established by single cell cloning of the positive clones. Unexpectedly the best producing cell lines were equally productive to the best CHO-pGB/IL-328 lines indicating that either the short 5' untranslated region of the mRNA has no effect of the production of hIL-3 or that the CMV enhancer/promoter is more effective in the CHO cells.

The IL-3 present in the conditioned media of the established cell lines showed high activity in the biological assays described in WO 88/04691.

### Example 3

### Expression of Human IL-3 in Human cells using a vector containing a modified BPV genome

The BPV genome encodes 3 proteins with sequences of the E2 open reading frame called E2-ta, E2-tr and E8/E2 respectively (Lambert et al., Annu. Rev. Genet. (1988), 22, 235-258). The ATG codon at position 3091-3093 may be changed into either an GCG (Ala) or an ACG (Thr) thereby on the one hand prohibiting the translation initiation of the E2-tr protein and on the other hand substituting Met¹⁶² in E2-ta with Ala¹⁶² or Thr¹⁶². The BPV genome in pGB/IL-328 and pGB/IL-330 may be exchanged by the E2-tr mutant BPV genomes. The equivalents of pGB/IL-328 are designated pGB/IL-331 (Ala) and pGB/IL-332 (Thr). The equivalents of pGB/IL-330 are designated pGB/IL-333 (Ala) and pGB/IL-334 (Thr). The new expression vectors can be introduced into C127 and CHO cells as described. The level of hIL-3 production is expected to be substantially raised in both cell types with respect to the C127-pGB/IL-328, CHO-pGB/IL-328 and C127-pGB/IL-330, CHO-pGB/IL-330 combinations respectively.

The IL-3 present in the conditioned media of the established cell lines will show high activity in the biological assays described in WO 88/04691.

### Example 4

### Purification of Human IL-3 obtained from expression in bacteria

### a. Bacillus licheniformis

The following procedure was followed to obtain highly purified and homogeneous hIL-3 from Bacillus licheniformis T9 (WO 88/04691) transformants. The transformants contained plasmid pGB/IL-322. However, pGB/IL-341 was also used in this procedure.

Cell free medium from B. licheniformis T9 was brought to 1M (NH₄)₂SO₄ and adjusted to pH 7.0 with NaOH and loaded on a column of Fractogel® TSK-butyl 650C, equilibrated in 1M (NH₄)₂SO₄ in 10mM Tris-HCL buffer, pH 7.0. 1mM Phenylmethylsulfonylfluoride (PMSF) was used as a proteinase inhibitor.

Whereas most of the protein was found in the run-through fractions, hIL-3 was adsorbed to the column. After extensive washing of this column with the same buffer, hIL-3 was eluted in between a gradient from 1M to OM (NH₄)₂SO₄ in 10mM Tris-HCl, pH 7.0. The hIL-3 enriched fractions were concentrated by (NH₄)₂SO₄ precipitation at 70% saturation. Subsequently, the precipitate obtained was desalted up to a conductivity of 0.7 mS or less and run on a column of Fractogel® TSK-DEAE 650M that was equilibrated in the same buffer (Tris-HCl, pH 7.8). Whereas hIL-3 activity was found in the run-through fractions, most of the contaminating proteins was bound to the column. The hIL-3 enriched run-through fractions were concentrated (either by adsorption and separation on a small column of Fractogel® TSK-butyl 650C as described above or by (NH₄)₂SO₄-precipitation) and purified to homogeneity by gel filtration on Biogel® A (0.5M; 100-200 mesh) with 200 mM NaCl in 20 mM Tris-HCl, pH 7.0, as the running buffer (Sephacryl® S100 HR also turned out to be an efficient matrix for gel filtration of hIL-3). This three-step purification procedure; 1) hydrophobic interaction, 2) anion-exchange chromatography and 3) gel filtration, resulted in a hIL-3 preparation that is free from non-hIL-3-like protein, as was determined by SDS-polyacrylamide gel-electrophoresis (SDS-PAGE) followed by silverstaining and by immunoblotting using anti-hIL-3 antibodies.

If hIL-3 degradation products were present in the starting medium obtained from B. licheniformis T9, selective removal of these proteolytic degradation products from the fraction interest was carried out by the following purification procedures:
1. hIL-3-enriched fractions derived from step 1 of the purification procedure were concentrated as described above, adjusted to pH 7.8 and brought to a conductivity of 0.7mS. hIL-3 was then loaded on a column of Q Sepharose® Fast-Flow (50x5cm) that was equilibrated in a Tris buffer with identical pH and conductivity (with a flow of about 600ml/h). In this purification step, the volume of the concentrated solution with hIL-3 that was loaded did comprise just 6-8% of the total column volume. All active forms of hIL-3 were found in the run-through fractions. The major form of hIL-3 was separated from other hIL-3-like smaller proteins that were also positive in western blotting using antihuman IL-3 antibodies. When contaminating hIL-3 degradation products were still present in the peak fraction of interest, this isolation procedure was repeated. Amino-terminal sequence analysis of the first 30 amino acids of the major form of hIL-3 revealed a sequence that is identical with the mature protein.
2. Chromatography on hydroxylapatite turned out to be a potent tool for the separation of several B. licheniformis T9 hIL-3 degradation products. hIL-3 was loaded on a Biogel® hydroxylapatite column in 10mM NaH₂PO₄, 0.01mM CaCl₂, 0.02% NaH₃, pH 6.8 and was eluted from the column with a gradient from 10-350mM NaH₂PO₄ in the same solution. This method was effective on both an analytical as well as a preparative scale.
3. hIL-3 degradation products from B. licheniformis T9 could also be separated by means of chromatofocussing on the polybuffer exchanger pBE94. By the application of a pH-gradient onto the column, different hIL-3 degradation products were eluted between pH 6.5 and pH 8.5.

In a similar isolation procedure based on hydrophobic interaction of hIL-3 from B. licheniformis on octyl-Sepharose® instead of Fractogel® TSK-butyl, hIL-3 was eluted by the application of a gradient from 0% (v/v)-100%(v/v) ethylene glycol which illustrates the stronger binding characteristics for this matrix.

All hIL-3 preparations obtained by the isolation procedures described above were positive in an AML-assay described in WO 88/04691.

### b. E. coli

For the isolation of a lacZ/hIL-3 fusion product that was intracellularly expressed in Escherichia coli (pGB/IL-302) and stored in inclusion bodies, the cells were lysed in a Tris-buffer, pH 8.0, containing 1%(v/v) octanol, 1%(w/v) Tx100, 0.1%(w/v) EDTA and 0.025%(w/v) Lysozyme. 1mM PMSF was used as a proteinase inhibitor.

After an extra incubation with DNAse and Mg²⁺, the crude inclusion bodies were centrifuged (10 min., 4000g) and washed in the same Tris buffer as mentioned above. After an extra centrifugation procedure (30 min., 10.000g), inclusion body derived protein was solubilized in 8M urea in Tris buffer under reducing conditions (5mM dithiothreitol, DTT). Finally, centrifugation (30 min., 27.000g) resulted in a supernatant with most of the hIL-3 that was originally present in the inclusion bodies. This solution of hIL-3 was adjusted to pH 5.0 and loaded on a Fractogel® TSK-CM 650M column, equilibrated in a buffer containing 8M urea, 5mM DTT, pH 5.0. IL-3 was eluted from this column by a linear salt gradient from 0-300mM NaCL in the same buffer. IL-3 from Escherichia coli, renaturated and solubilised in urea and partly purified by ion-exchange chromatography, was positive in an AML assay.

The above purification procedure was shown to be highly effective for the isolation of interleukin-3 originating from other primates. Rhesus 1L-3 was successfully expressed and secreted by Bacillus licheniformis. A cell free filtrate of Bacillus licheniformis was applied onto a TSK Butyl column as described above. Rhesus IL-3 was eluted from this column by using a gradient of (NH₄)₂SO₄ (1-OM). The IL-3 containing fractions were concentrated and subjected to DEAE Sepharose® Fast Flow chromatography as described for human IL-3 on Q-Sepharose® Fast Flow. The unbound fraction of Rhesus IL-3 was collected and tested for its purity by polyacrylamide gel electrophoresis and western blotting.

### Example 5

### Purification of human IL-3 obtained from expression in yeasts

After centrifugation of a culture of Kluyveromyces lactis transformants containing pGB/IL-316 (WO 88/04691) the hIL-3-containing supernatant was filtrated for residual cell removal, brought to a concentration of 1M (NH₄)₂SO₄ and adjusted to pH 7.0. (1mM PMSF was used as a proteinase inhibitor). This solution was loaded on a column of octyl-Sepharose® equilibrated in 1M(NH₄)₂SO₄ in phosphate buffer, pH 7.0. Whereas most of the protein was found in the run-through fractions hIL-3 was adsorbed to the column. After a washing procedure with the same buffer, hIL-3 was partly released from the column with a gradient from 1M - OM (NH₄)₂SO₄ in phosphate buffer, pH 7.0.

The major part of hIL-3 which was still adsorbed to the column was eluted by the application of a gradient from 0%(v/v)-100%(v/v) ethylene glycol. The technique of hydrophobic interaction of hIL-3 on octyl-Sepharose® (or Fractogel® TSK-butyl 650C) turned out to be a highy efficient first isolation procedure. This technique followed by ion-exchange chromatography and gel filtration resulted in a homogenous preparation of hIL-3 from Kluyveromyces lactis.

In a similar procedure using the medium from Saccharomyces cerevisiae transformants containing pGB/IL-316, hIL-3 was also eluted from octyl-Sepharose® with a gradient from 0%(v/v)-100%(v/v) ethylene glycol.

In an alternative procedure, isolation of hIL-3 from the cell free medium of a culture of Kluyveromyces lactis started with (NH₄)₂SO₄ precipitation (at 75% saturation), 5 this method resulted in precipitation of most hIL-3 included in just a minor part of the total amount of protein that was originally present. The collected precipitate was resuspended in, and dialysed against, phosphate buffer. The hIL-3 containing solution was adjusted to pH 5.0 with NaOH and loaded on a Fractogel® TSK-CM 650M cation-exchange column, equilibrated in the same buffer. Whereas all hIL-3 was adsorbed to the column, most of the contaminating protein was found in the run-through fractions. Human IL-3 was eluted from the column by the application of a gradient from 0mM-300mM NaCl in the same buffer.

Fractionated hIL-3 obtained by this two step procedure was close to homogeneity. As a result of the nature of glycosylation, inherent when using Kluyveromyces lactis as the expression system, hIL-3 purified to homogeneity, turned out to be composed of different forms of the protein with respect to their molecular weights. All hIL-3 preparations obtained from the above mentioned yeast expression systems were positive in an AML-assay.

### Example 6

### Purification of human IL-3 obtained from expression in Mammalian cells

hIL-3 obtained from a mammalian cell culture was purified from the medium by using similar methods as described for hIL-3 obtained from expression in B. licheniformis (Example 4). Medium from transformed C127-cells containing pGB/IL-328 was brought to 1M (NH₄)₂SO₄, adjusted to pH 7.0 with NaOH and loaded on a Fractogel® TSK-butyl 650C column (1mM PMSF was used as a proteinase inhibitor). hIL-3 enriched fractions (with about 10% of total protein that was loaded on the column) were eluted with a gradient from 1M to 0M (NH₄)₂SO₄ in 10mM Tris-HCl, pH 7.0. These fractions were concentrated by ammonium sulphate precipitation at 60% saturation. The protein precipitate was dissolved and dialysed against H₂O, after which 20mM Tris-HCl, pH 7.8, was added up to a conductivity of 1.4mS or less. The hIL-3 preparation was subsequently loaded on a Fractogel® TSK-DEAE 650M column. hIL-3 was partly found in the run-through fractions and was also eluted with a gradient from 0-200mM NaCl. Most of the contaminating protein was bound to the column and was eluted after the hIL-3 fractions.

This two step procedure combined with (NH₄)₂SO₄ precipitation resulted again in a concentrated hIL-3 preparation in just 1% of the total amount of protein that was originally present in the starting material. This preparation was positive in an AML-assay (WO 88/04691).

### Example 7

### Large scale purification of hIL-3 from Bacillus licheniformis T9 (pGB/IL-322)

hIL-3 was purified from B. licheniformis T9 (pGB/IL-322) using the following four step large-scale purification scheme.

### Step one: Hydrophobic interaction chromatography

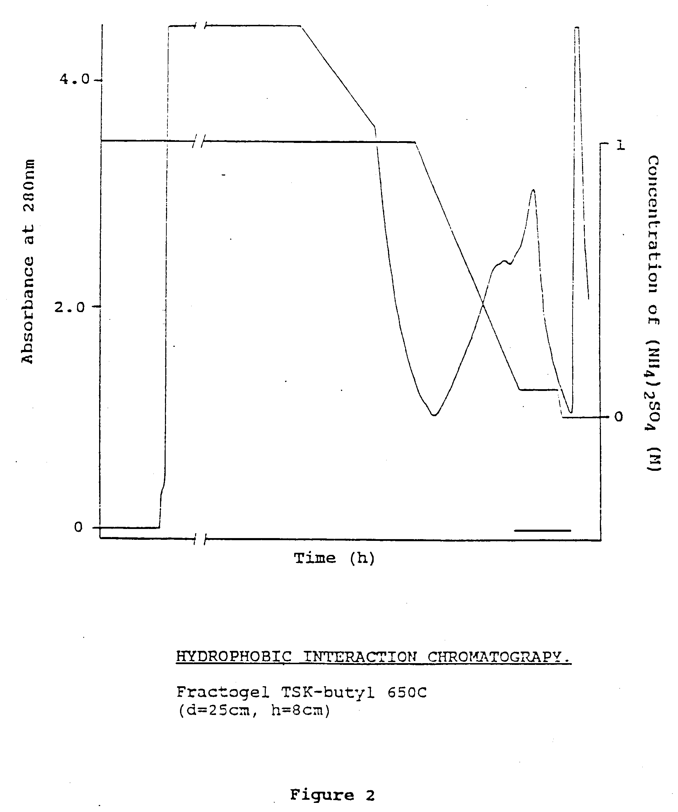

The cell-free supernatant derived from 6 fermentation runs of Bacillus licheniformis T9 (WO 88/04691), having a total volume of 48 liter, was brought to 1M (NH₄)₂SO₄, pH = 7.0. Human IL-3 was adsorbed on a Fractogel® TSK-butyl 650C column (dxh = 25x8cm) equilibrated with 1M (NH₄)₂SO₄ in Tris, pH = 7.0 (q = 8 l/h). The column was extensively washed with the same buffer. HIL-3 was eluted with a linear salt gradient from 1.0 - 0.1M (NH₄)₂SO₄ in 10mM Tris, pH=7.0 (Figure 2). The hIL-3 enriched fractions were further concentrated by (NH₄)₂SO₄-precipitation at 60% saturation and the precipitate was desalted by dialysis (Spektra/Por, cut off 3.500D) against Milli-Q water up to a conductivity of 0.70mS or less.

### Step two: Anion-exchange chromatography (first time)

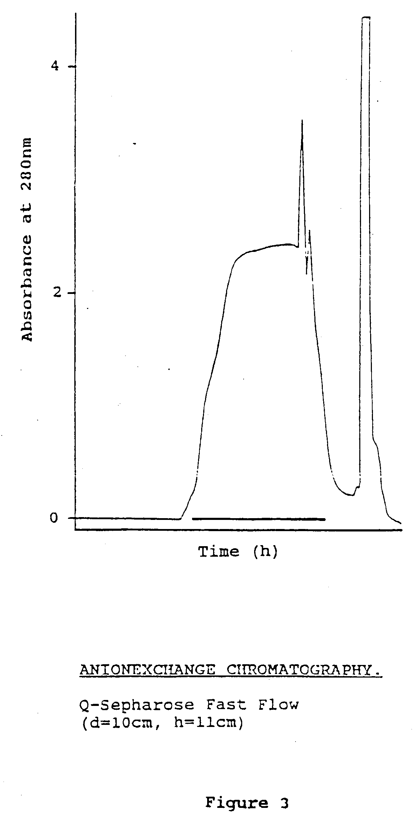

The hIL-3 enriched solution was brought to pH = 7.8, using solid Tris, and a to a final conductivity of less than 0.70mS. Subsequently it was brought on a Q-Sepharose® Fast Flow column (dxh = 10x11cm, q = 3 l/h) equilibrated in a Tris buffer, pH = 7.8 and a conductivity of less than 0.70mS. HIL-3 was collected in the run-through fraction, most of the contaminating protein was bound to the column (Figure 3). HIL-3 was concentrated to 25-30 mg/ml by ultrafiltration (Amicon, ym5-filter).

### Step three: Anion-exchange chromatography (second time)

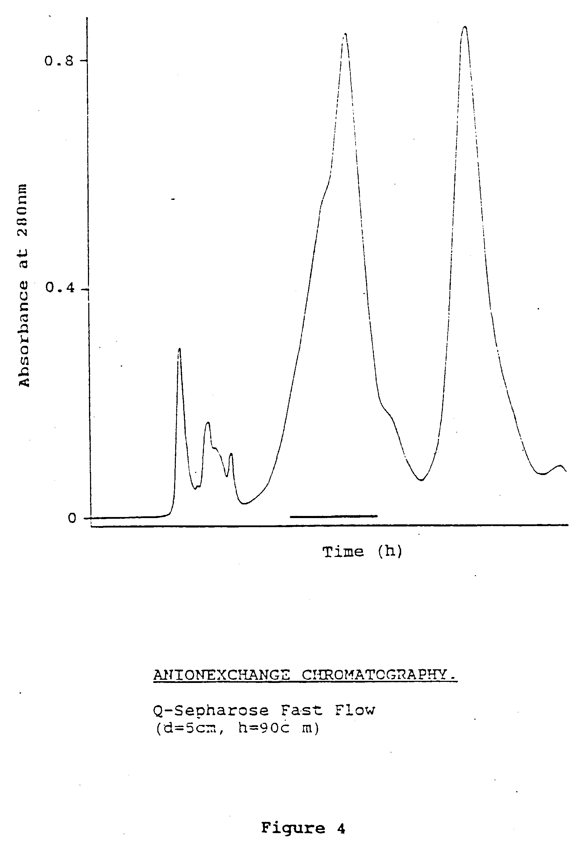

Sixty milliliters (containing 1.5 -1.8g hIL-3) of the solution obtained from the previous step was run on a Q-Sepharose® Fast Flow column (dxh = 5x90cm, q = 0.3 l/h). Several peaks with hIL-3 activity were detected in the run-through fractions (Figure 4). Thus the hIL-3 protein fraction of interest was separated from hIL-3 degradation products. The hIL-3 was concentrated as described above.

### Step four : Gel filtration chromatography

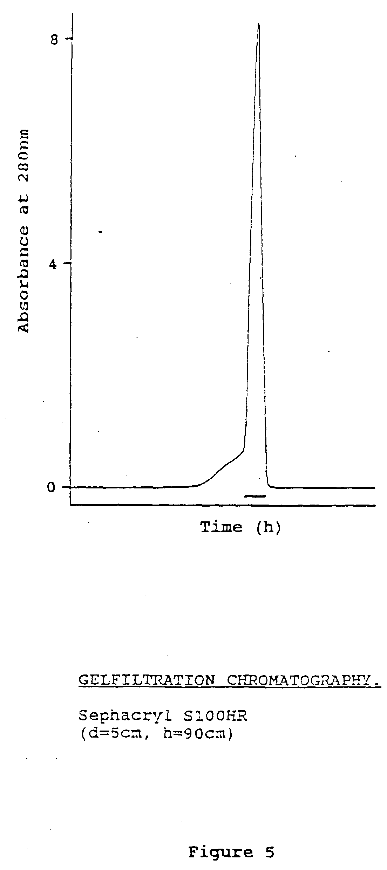

An amount of about 0.9g hIL-3 (obtained from the above steps) was brought on a Sephacryl® S100 HR column (dxh = 5x90cm, q = 0.15 l/h) equilibrated in 10mM NaH₂PO₄, 140mM NaCl, pH = 7.0. The hIL-3 was collected in a single peak which was separated from a small amount of hIL-3 aggregates eluting just before the hIL-3 (Figure 5).

HIL-3 obtained by this purification procedure was free from detectable amounts of contaminating protein and nucleic acid. The solution containing hIL-3 was desalted and subsequently concentrated by ultrafiltration to the appropriate concentration.

### Example 8

### Characterization of recombinant interleukin-3

### a. Plasma Desorption Mass Spectrometry of purified IL-3

Recombinant hIL-3, derived from pGB/IL-341 and purified as described in Example 4, was characterized by peptide mapping and mass spectrometry.

Subsequently hIL-3 was proteolytically degraded by various specific proteases, followed by separation of the peptides obtained by reversed phase HPLC on a C18-column. The hIL-3-peptides were analysed by Plasma Desorption Mass Spectrometry. The molecular weights of the hIL-3-peptides obtained could all be explained on the basis of the sequence and the known enzyme specificities. By this means the primary sequence information of the purified protein was shown to be identical with that of the native protein (129 residues for pGB/IL-341). It can be concluded that based on C-terminal peptide analysis, the purification process described results in a product consisting of recombinant IL-3 with a purity of more than 98%.

### b. In vivo effect of hIL-3 in chimpanzees

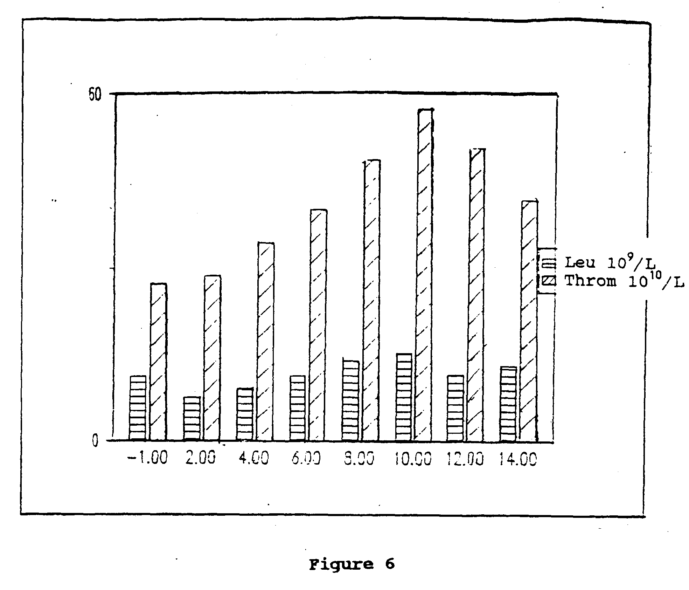

The recombinant hIL-3 produced by B. licheniformis cells was purified according to the procedure described in Example 4. Subsequently the IL-3 solution was diluted in pyrogen-free water to give the required final concentration. Chimpanzees were injected subcutaneously with this solution. Administration of 30 µg of IL-3 per kg per day for a period of seven days resulted in an increase of thrombocytes and a tendency to leucocytosis (Figure 6). This surprising result of IL-3 activity as a single agent was unanticipated and may indicate a clinical application for IL-3 in thrombocytopenia.

## Claims

1. A method of obtaining a substantially pure protein having primate IL-3 activity from a crude protein product obtained from a recombinant cell expressing a DNA sequence corresponding to a primate IL-3, comprising:
(a) performing hydrophobic interaction chromatography on said protein product and collecting fractions containing protein having IL-3 activity; and,
(b) performing ion-exchange chromatography on the fractions from step (a), and collecting fractions containing protein having IL-3 activity; and,
(c) performing gel filtration chromatography on the protein containing fractions of step (b), and collecting the purified protein.

2. A method according to claim 1 wherein the primate IL-3 is human IL-3.

3. The method of claim 1 or 2 wherein said recombinant cell is a Bacillus cell containing a DNA sequence corresponding to IL-3.

4. The method of claim 3 wherein said Bacillus cell is selected from the group consisting of B. licheniformis T9 containing pGB/IL-322, B. licheniformis T9 containing pGB/IL-326 or B. licheniformis T9 containing pGB/IL-341.

5. The method of claim 1 or 2 wherein said recombinant cell is a yeast cell containing a DNA sequence corresponding to IL-3.

6. The method of claim 5 wherein said yeast cell is selected from the group consisting of K. lactis containing pGB/IL-316 and S. cerevisiae containing pGB/IL-316.

7. The method of claim 1 or 2 wherein said recombinant cell is a mammalian cell containing a DNA sequence corresponding to IL-3 on a vector containing part of the BPV genome.

8. The method of claim 7 wherein said mammalian cell is selected from the group consisting of C127 cells containing pGB/IL-328, CHO cells containing pGB/IL-328, FR3T3 cells containing pGB/IL-328, C127 cells containing pGB/IL-330 and CHO cells containing pGB/IL-330.

9. A method of obtaining a substantially pure protein having primate IL-3 activity according to claim 1 characterized in that the ion-exchange chromatography step is preceded by the following steps :
(a) precipitating said protein using (NH₄)₂SO₄; and
(b) resuspending said protein-containing precipitate.

10. A substantially pure protein having primate IL-3 activity with a purity higher than 98% as determined by C-terminal peptide mapping in combination with mass spectrometry.

## Patentansprüche

1. Verfahren zum Erhalten eines im wesentlichen reinen Proteins mit IL-3-Aktivität eines Primaten aus einem rohen Proteinprodukt, das aus einer Rekombinantenzelle erhalten worden ist, die eine DNS-Sequenz entsprechend einem IL-3 eines Primaten exprimiert, mit folgenden Stufen:
(a) Durchführen einer Chromatographie mit hydrophober Wechselwirkung, angewandt auf das genannte Proteinprodukt, und Sammeln von Fraktionen, die Protein mit IL-3-Aktivität enthalten,
(b) Durchführen einer Ionenaustauschchromatographie mit den Fraktionen aus der Stufe (a) und Sammeln von Fraktionen, die ein Protein mit IL-3-Aktivität enthalten sowie
(c) Durchführen einer Gelfiltrationschromatographie mit dem Protein, das Fraktionen der Stufe (b) enthält, und Sammeln des gereinigten Proteins.

2. Verfahren nach Anspruch 1, worin das IL-3 eines Primaten humanes IL-3 ist.

3. Verfahren nach Anspruch 1 oder 2, worin die Rekombinantenzelle eine Bacilluszelle ist, die eine DNS-Sequenz entsprechend IL-3 enthält.

4. Verfahren nach Anspruch 3, worin die Bacilluszelle aus der Gruppe ausgewählt ist, die aus B. licheniformis T9, enthaltend pGB/IL-322, B. licheniformis T9, enthaltend pGB/IL-326, oder B. licheniformis T9, enthaltend pGB/IL-341, besteht.

5. Verfahren nach Anspruch 1 oder 2, worin die Rekombinantenzelle eine Hefezelle ist, die eine DNS-Sequenz entsprechend IL-3 enthält.

6. Verfahren nach Anspruch 5, worin die Hefezelle aus der Gruppe ausgewählt ist, die aus K. lactis, enthaltend pGB/IL-316, und S. cerevisiae, enthaltend pGB/IL-316, besteht.

7. Verfahren nach Anspruch 1 oder 2, worin die Rekombinantenzelle eine Säugetierzelle ist, die eine DNS-Sequenz entsprechend IL-3 auf einem Vector enthält, der einen Teil des BPV-Genoms enthält.

8. Verfahren nach Anspruch 7, worin die Säugetierzelle aus der Gruppe ausgewählt ist, die aus C127-Zellen, enthaltend pGB/IL-328, CHO-Zellen, enthaltend pGB/IL-328, FR3T3-Zellen, enthaltend pGB/IL-328, C127-Zellen, enthaltend pGB/IL-330, und CHO-Zellen, enthaltend pGB/IL-330, besteht.

9. Verfahren zum Erhalten eines im wesentlichen reinen Proteins mit IL-3-Aktivität eines Primaten gemäß Anspruch 1, dadurch gekennzeichnet, daß der Stufe der Ionenaustauschchromatographie die folgenden Stufen vorausgehen:
(a) Ausfällen des Proteins unter Verwendung von (NH₄)₂SO₄ und
(b) erneutes Suspendieren des proteinhaltigen Präzipitats.

10. Im wesentlichen reines Protein mit IL-3-Aktivität eines Primaten mit einer Reinheit von über 98 %, bestimmt durch Darstellen von C-terminalem Peptid in Kombination mit der Massenspektrometrie.

## Revendications

1. Procédé d'obtention d'une protéine sensiblement pure, ayant une activité d'IL-3 de primate, à partir d'un produit protéique brut obtenu à partir d'une cellule recombinée exprimant une séquence d'ADN correspondant à une IL-3 de primate, comprenant :
(a) la réalisation d'une chromatographie à interactions hydrophobes du produit protéique et la collecte des fractions contenant la protéine ayant une activité d'IL-3;
(b) la réalisation d'une chromatographie échangeuse d'ions sur les fractions de l'étape (a) et la collecte des fractions contenant la protéine ayant une activité d'IL-3, et
(c) la réalisation d'une chromatographie de filtration sur gel sur les fractions contenant la protéine de l'étape (b) et la collecte de la protéine purifiée.

2. Procédé suivant la revendication 1, dans lequel l'IL-3 de primate est de l'IL-3 humaine.

3. Procédé suivant la revendication 1 ou 2, dans lequel la cellule recombinée est une cellule Bacillus contenant une séquence d'ADN correspondant à l'IL-3.

4. Procédé suivant la revendication 3, dans lequel la cellule Bacillus est choisie dans le groupe consistant en B. licheniformis T9 contenant pGB/IL-322, B. licheniformis T9 contenant pGB/IL-326 ou B. licheniformis T9 contenant pGB/IL-341.

5. Procédé suivant la revendication 1 ou 2, dans lequel la cellule recombinée est une cellule de levure contenant une séquence d'ADN correspondant à l'IL-3.

6. Procédé suivant la revendication 5, dans lequel la cellule de levure est choisie dans le groupe consistant en K. lactis contenant pGB/IL-316 et S. cerevisiae contenant pGB/IL-316.

7. Procédé suivant la revendication 1 ou 2, dans lequel la cellule recombinée est une cellule de mammifère contenant une séquence d'ADN correspondant à l'IL-3 sur un vecteur contenant une partie du génome BPV.

8. Procédé suivant la revendication 7, dans lequel la cellule de mammifère est choisie dans le groupe consistant en les cellules C127 contenant pGB/IL-328, les cellules CHO contenant pGB/IL-328, les cellules FR3T3 contenant pGB/IL-328, les cellules C127 contenant pGB/IL-330 et les cellules CHO contenant pGB/IL-330.

9. Procédé d'obtention d'une protéine sensiblement pure, ayant une activité d'IL-3 de primate, suivant la revendication 1, caractérisé en ce que l'étape de chromatographie échangeuse d'ions est précédée des étapes suivantes :
(a) précipitation de la protéine au moyen de (NH₄)₂SO₄, et
(b) remise en suspension du précipité contenant la protéine.

10. Protéine sensiblement pure, ayant une activité d'IL-3 de primate, avec une pureté supérieure à 98%, déterminée par établissement de la carte du peptide C-terminal en association avec la spectrométrie de masse.
